# EUROPEAN PATENT SPECIFICATION

(11) **EP 3 409 283 B1**
(45) Date of publication and mention of the grant of the patent: **02.03.2022**
(21) Application number: 17744399.1
(22) Date of filing: 27.01.2017
(51) Int. Cl.: A61K 35/36, A61K 35/76, A61P 21/00, A61K 9/00

(54) **RABBIT SKIN EXTRACT FOR PROPHYLAXIS OR TREATMENT OF MUSCLE DAMAGE**
KANINCHENHAUTEKSTRAKT ZUR PROPHYLAXE ODER BEHANDLUNG VON MUSKELBESCHÄDIGUNG
EXTRAIT DE PEAU DE LAPIN POUR LA PROPHYLAXIE ET/OU LE TRAITEMENT DES LÉSIONS MUSCULAIRES

(30) Priority: 29.01.2016 JP 2016016463
(43) Date of publication of application: 05.12.2018
(73) Proprietor: National University Corporation Chiba University, Chiba-shi, Chiba 263-0022 (JP); Nippon Zoki Pharmaceutical Co., Ltd., Chuo-ku Osaka-shi, Osaka 541-0046 (JP)
(72) Inventor: INAGE, Kazuhide, Chiba-shi Chiba 260-8670 (JP); TAKAHASHI, Kazuhisa, Chiba-shi Chiba 260-8670 (JP); OHTORI, Seiji, Chiba-shi Chiba 260-8670 (JP); ORITA, Sumihisa, Chiba-shi Chiba 260-8677 (JP)
(74) Representative: Abel & Imray LLP
(86) International application number: PCT/JP2017/002973
(87) International publication number: WO 2017/131169

(56) References cited:
- EP-A1- 1 865 067
- WO-A1-2010/131712
- WO-A1-2014/057995
- WO-A2-2012/051173
- JP-B2- 3 852 786
- US-A- 6 165 515
- US-A1- 2013 028 982
- YUKO NAITO et al.: "Lack of Neu5Gc Expression Contributes to the Severity of Duchenne Muscular Dystrophy in Humans", Trends in Glycoscience and Glycotechnology, vol. 23, no. 132 2011, pages 194-196, XP055535405, Retrieved from the Internet: URL:DOI https://doi.org/10.4052/tigg.23.194

## Description

### [Technical Field]

The present invention relates to a novel medical use of an extract from inflamed tissues inoculated with vaccinia virus and, more particularly, it relates to a preventive or therapeutic agent for muscle injury containing an extract from inflamed tissues inoculated with vaccinia virus as an active ingredient.

### [Background Art]

Muscle injury (i.e., injuries of muscle) is the injury of muscle fibers. Muscle injury is graded into three stages, i.e., stage I (slight injury of muscle fiber), stage II (partial rupture of muscle fiber) and stage III (complete rupture of muscle fiber), depending on the severity of the injury.

When muscle injury occurs, firstly vasodilatation and permeability enhancement are caused, and then a reaction of which the sign is enlargement or warmth, i.e., an inflammatory reaction, is induced. In an inflammatory reaction, inflammatory cells such as neutrophils or macrophages which are one type of leukocytes are infiltrated into an injury site to phagocytize necrotic muscle fibers and, at the same time, secrete cytokines that can proliferate muscle satellite cells or the like. The muscle satellite cells are activated rapidly in the injury site and are converted to myoblasts. The myoblasts are proliferated repeatedly, and are then fused to each other. In this manner, the myoblasts are differentiated into myotube cells. The myotube cells finally become mature into muscle fibers to finish the repair of the muscles (spontaneous remission). The period needed for the completion of the repair of muscles is normally about 3 weeks to 1 month in the case where the level of muscle injury is stage II.

As the method for treating muscle injury, a conservative treatment, a surgical treatment and the like can be mentioned. Among these methods, although a surgical treatment is usually employed when muscle injury is the complete rupture of muscles, a conservative therapy is generally employed.

However, in a conservative therapy which greatly relies on spontaneous remission, there is a risk such that a patient is forced to have a difficult life for a long period to some extent, as mentioned above. Therefore, a treatment method that can repair muscle injury earlier has been demanded.

The extract from inflamed tissues inoculated with vaccinia virus as an active ingredient in the preventive or therapeutic agent for muscle injury of the present invention (hereinafter referred to as "the medicament of the present invention") is disclosed to have the following effects: an analgesic effect, sedative effect, anti-stress effect and anti-allergic effect (see Patent Document 1); an immunostimulating effect, anti-cancer effect and cirrhosis inhibitory effect (see Patent Document 2); a treatment effect against idiopathic thrombocytopenic purpura (see Patent Document 3); a treatment effect against postherpetic neuralgia, brain edema, dementia, spinocerebellar degeneration and the like (see Patent Document 4); a treatment effect against Raynaud syndrome, diabetic neuropathy, sequelae of subacute myelo-optico-neuropathy and the like (see Patent Document 5); a kallikrein production inhibitory effect and peripheral circulatory disorder improving effect (see Patent Document 6); a bone atrophy improving effect (see Patent Document 7); a nitric oxide production inhibitory effect effective for the treatment of sepsis and endotoxic shock (see Patent Document 8); a treatment effect against osteoporosis (see Patent Document 9); a treatment effect against AIDS based on a Nef action inhibitory effect and chemokine production inhibitory effect (see Patent Documents 10 and 11); a treatment effect against ischemic disorders such as cerebral infarction (see Patent Document 12); a treatment effect against fibromyalgia syndrome (see Patent Document 13); and a treatment effect against infections (see Patent Document 14); a prophylactic or alleviating effect for a peripheral nerve disorder induced by an anti-cancer agent (see Patent Document 15); a treatment effect against chronic prostatitis, interstitial cystitis an/or urination disorders (see Patent Document 16); an effect of promoting production of neurotrophic factor such as BDNF (see Patent Document 17); an effect of promoting the synthesis of collagen and proteoglycan in chondrocytes (see Patent Document 18) and the like. However, as far as we know, it is not known that the extract from inflamed tissues inoculated with vaccinia virus as an active ingredient in the medicament of the present invention is effective for prevention or therapy for muscle injury.

### [Prior Art Documents]

### [Patent Documents]

Patent Document 1: Japanese Patent Laid-Open No. Sho-53-101515
Patent Document 2: Japanese Patent Laid-Open No. sho-55-87724
Patent Document 3: Japanese Patent Laid-Open No. Hei-1-265028
Patent Document 4: Japanese Patent Laid-Open No. Hei-1-319422
Patent Document 5: Japanese Patent Laid-Open No. Hei-2-28119
Patent Document 6: Japanese Patent Laid-Open No. Hei-7-97336
Patent Document 7: Japanese Patent Laid-Open No. Hei-8-291077
Patent Document 8: Japanese Patent Laid-Open No. Hei-10-194978
Patent Document 9: Japanese Patent Laid-Open No. Hei-11-80005
Patent Document 10: Japanese Patent Laid-Open No. Hei-11-139977
Patent Document 11: Japanese Patent Laid-Open No. 2000-336034
Patent Document 12: Japanese Patent Laid-Open No. 2000-16942
Patent Document 13: International Publication No. WO 2004/039383
Patent Document 14: Japanese Patent Laid-Open No. 2004-300146
Patent Document 15: International Publication No. WO2009/028605
Patent Document 16: International Publication No. WO2011/111770
Patent Document 17: International Publication No. WO2011/162317
Patent Document 18: International Publication No. WO2012/051173

### [Summary of the Invention]

### [Problem to be Solved by the Invention]

The object of the present invention is to provide a medicament that is effective for the prevention or treatment of muscle injury and is highly safe.

### [Means for Solving the Problems]

The present inventors have made extensive and intensive studies on a medicinal treatment for muscle injury for which an effective treatment method has been still demanded. As a result, it has been found that an extract from inflamed tissues inoculated with vaccinia virus has an excellent repair promotive effect on muscle injury. This finding leads to the accomplishment of the present invention.

According to the present invention, it becomes possible to greatly shorten the period required for the healing of muscle injury by repair.

### [Advantages of the Invention]

An extract from inflamed tissues inoculated with vaccinia virus has an excellent pharmacological effect to promote the repair of muscle injury.

Thereby, the extract can decrease the extent of muscle injury when administered before the onset of the muscle injury, and can heal muscle injury in an earlier stage when administered after the onset of the muscle injury.

Therefore, a medicament of the present invention which contains the extract as an active ingredient is highly useful as a preventive or therapeutic agent for muscle injury. In particular, the medicament of the present invention has very little disadvantages such as side effects, and is therefore highly safe.

### [Mode for Carrying Out the Invention]

The present invention relates to an extract from inflamed tissues of rabbit skin inoculated with vaccinia virus as an active ingredient, for use in treating a muscle injury or promoting a muscle injury repair. The extract is obtainable by crushing said inflamed tissue, adding an extraction solvent to remove tissue fragments, deproteinising the solution, adsorbing the deproteinised solution onto an adsorbent, and eluting the extract. An extract from inflamed tissues inoculated with vaccinia virus has an effect to promote the repair of muscle injury. Therefore, the medicament of the present invention may be used as a muscle injury repair promoter.

The muscle in muscle injury to be prevented or treated by the medicament of the present invention is not particularly limited, as long as the muscle has a part in which muscle injury has been occurred or a part in which muscle injury may be occurred. Examples of the muscle include quadriceps femoris muscle, hamstrings, paraspinal muscle and gastrocnemius muscle. The cause of muscle injury is not particularly limited, and muscle injury associated with surgical invasion is also a target to be prevented or treated with the medicament of the present invention.

As for the extract from inflamed tissues inoculated with vaccinia virus, there are various reports on physiological active substances produced in the inflamed tissues inoculated with vaccinia virus, the method for extracting the substances from the diseased tissues, the pharmacological activities and the like as mentioned above (for example, Patent Documents 1 to 18).

Furthermore, a preparation of an extract from inflamed skins of rabbits inoculated with vaccinia virus is a commercially available pharmaceutical product which is available for the medicinal agent of the present invention. The preparation, as described in pages 3525 to 3527 of "Drugs in Japan, Ethical Drugs" (2016, edited and published by Japan Pharmaceutical Information Center), contains non-proteinous active substances extracted and separated from the inflamed skin tissues of rabbits inoculated with vaccinia virus. The preparation is known to be effective against low back pain, cervicobrachial syndrome, symptomatic neuralgia, periarthritis scapulohumeralis, osteoarthritis, itchiness accompanied with skin diseases (eczema, dermatitis, urticaria), allergic rhinitis, sequelae of subacute myelo-optico-neuropathy such as coldness, paresthesia and pain, postherpetic neuralgia and the like. The preparation is approved as an ethical drug in the form of hypodermic, intramuscular and intravenous injection products and of tablets and is commercially available.

Hereinafter, the process for producing the extract of inflamed tissues of rabbit skin inoculated with vaccinia virus as an active ingredient in the medicinal agent of the present invention and the like will be described.

The extract from inflamed tissues inoculated with vaccinia virus used in the medicament of the present invention can be obtained by the following manner: inflamed tissues inflamed by the inoculation with vaccinia virus is crushed; an extraction solvent is added to remove the tissue fragments; then deproteinization is carried out; the deproteinized solution is adsorbed onto an adsorbent; and then the active ingredient is eluted.

As to basic extracting steps for the present extract, the following steps are used for example.
(A) Inflamed skin tissues of rabbits by the intradermal inoculation with vaccinia virus are collected, and the inflamed tissues are crushed. To the crushed tissues an extraction solvent such as water, phenol water, physiological saline or phenol-added glycerin water is added to conduct an extracting treatment for several days. Then, the mixture is filtrated or centrifuged to give a crude extract (filtrate or supernatant) wherefrom tissue fragments are removed.
(B) The crude extract obtained in (A) is adjusted to acidic pH, heated and then filtered or centrifuged to conduct a deproteinizing treatment. After that, the deproteinized solution is adjusted to basic pH, heated and then filtered or centrifuged to give a deproteinized filtrate or supernatant.
(C) The filtrate or the supernatant obtained in (B) is adjusted to acidic pH and adsorbed with an adsorbent such as activated carbon or kaolin.
(D) An extraction solvent such as water is added to the adsorbent obtained in (C), the mixture is adjusted to basic pH and the adsorbed component is eluted to give an extract from inflamed tissues inoculated with vaccinia virus.

The above is the basic steps and each of the steps will be more specifically illustrated as follows.

### About (A):

As for animals in order to obtain the inflamed tissues by the inoculation of vaccinia virus, the inflamed tissues are inflamed skin tissues of rabbits.

These inflamed tissues are collected, and washed and disinfected using a phenol solution, etc. These inflamed tissues are crushed and an extraction solvent in 1- to 5-fold thereof by volume is added to make an emulsified suspension. Here, the term "crush" means to finely break down into minces using a mincing machine or the like. As to the extraction solvent, there may be used distilled water, physiological saline, weakly acidic to weakly basic buffer, etc. and stabilizer such as glycerin, bactericidal/antiseptic agent such as phenol, salts such as sodium chloride, potassium chloride or magnesium chloride, etc. may be appropriately added thereto. At that time, it is also possible that the cell tissue is destroyed by a treatment such as freezing/melting, ultrasonic wave, cell membrane dissolving enzyme or surfactant so as to make the extraction easier. The resulting suspension is allowed to stand for 5 to 12 days. During that period, the suspension may be heated at 30 to 45°C with or without appropriate stirring. The resulting liquid is subjected to filtration, centrifugation, or the like to remove the tissue fragments whereupon a crude extract (filtrate or supernatant) is obtained.

### About (B):

The crude extract obtained in (A) is subjected to filtration, centrifugation or the like to remove tissue fragments, and then deproteinized. The deproteinization-treatment may be carried out by a known method which has been usually conducted and a method such as heating treatment, treatment with a protein denaturant (such as acid, base, urea, guanidine or an organic solvent including acetone), isoelectric precipitation or salting-out may be applied. After that, a common method for the removal of insoluble matters such as filtration using filter paper (such as cellulose or nitrocellulose), glass filter, Celite or Seitz filter, ultrafiltration or centrifugation is conducted to give a filtrate or a supernatant wherefrom the separated insoluble protein is removed.

### About (C)

The filtrate or supernatant obtained in (B) is adjusted to acidic or, preferably, to pH 3.5 to 5.5 with an acid such as hydrochloric acid, sulfuric acid or hydrobromic acid to conduct an operation of adsorbing with an adsorbent. Examples of the usable adsorbent include activated carbon and kaolin. An adsorbent is added to the extract followed by stirring or the extract is passed through a column filled with an adsorbent so that the active ingredient can be adsorbed with the adsorbent. When an adsorbent is added to the extract, the adsorbent with which the active ingredient is adsorbed can be obtained by means of filtration, centrifugation, etc. to remove the solution.

### About (D)

For elution (desorption) of the active ingredient from the adsorbent obtained in (C), an elution solvent is added to said adsorbent, elution is conducted at room temperature or with suitable heating, or with stirring, and then the adsorbent is removed by a common method such as filtration or centrifugation. As to the extraction solvent used therefore, there may be used a basic solvent such as water, methanol, ethanol, isopropanol or the like adjusted to basic pH or an appropriate mixed solvent thereof and preferably, water adjusted to pH 9 to 12 may be used. Amount of the extracting solvent may be appropriately set.

The extract (eluate) obtained in this manner may be properly prepared in a suitable form as a raw material for a formulation or a pharmaceutical formulation. For example, the solution may be adjusted to have nearly neutral pH to be a raw material for a formulation, and may be adjusted to have a desired concentration by concentration or dilution. In addition, for a formulation for injection, sodium chloride may be added to prepare a solution isotonic to physiological saline. Furthermore, the solution may be concentrated to dryness or freeze-dried to prepare a solid form available for the raw material of tablets or the like.

Examples of an administration method to a patient include oral and other administrations such as subcutaneous, intramuscular and intravenous administrations. The dose can be suitably determined depending on the type of extract from inflamed tissues inoculated with vaccinia virus. The dose that is approved in the commercially available preparation according to the "Drugs in Japan, Ethical Drugs" (page 3525) is principally 16 NU per day by oral administration and 3.6 to 7.2 NU per day by injection. However, the dose may be appropriately increased or decreased depending on the type of disease, degree of seriousness, individual difference in the patients, method of administration, period of administration and the like (NU: Neurotropin unit. Neurotropin unit is defined by ED₅₀ value of analgesic effect measured by a modified Randall-Selitto method using SART-stressed mice that are chronic stressed animals showing a lowered pain threshold than normal animals. One NU indicates the activity of 1 mg of analgesic ingredients in Neurotropin preparations when the ED₅₀ value is 100 mg/kg of the preparation).

Hereinafter, examples of methods for producing an extract from inflamed tissues inoculated with vaccinia virus as well as pharmacological tests concerning novel pharmacological activity of the extract, that is, the repair promotive effect on muscle injury, are described. The present invention is not intended to be limited to the descriptions in Examples.

### [Examples]

### Example 1

Skins of healthy adult rabbits were inoculated with vaccinia virus. The inflamed skins were removed and crushed, and to the crushed skins, phenolated water was added. Then, the mixture was filtered under pressure, and the obtained filtrate was adjusted to pH 5 with hydrochloric acid, and then heated at 90 to 100°C for 30 minutes. After deproteinization by filtration, the filtrate was adjusted to pH 9 with sodium hydroxide, further heated at 90 to 100°C for 15 minutes, and then filtered. The filtrate was adjusted to about pH 4.5 with hydrochloric acid, and 2% activated carbon was added. The mixture was stirred for 2 hours and then centrifuged. To the collected activated carbon, water was added. The mixture was adjusted to pH 10 with sodium hydroxide, stirred at 60°C for 1.5 hours, and then centrifuged and filtered to obtain a supernatant. To the collected activated carbon, water was added again. The mixture was adjusted to pH 11 with sodium hydroxide, stirred at 60°C for 1.5 hours, and then centrifuged to obtain a supernatant. The two supernatants were combined and neutralized with hydrochloric acid to obtain an extract from inflamed skins of rabbits inoculated with vaccinia virus.

### Example 2

Skins of healthy adult rabbits were inoculated with vaccinia virus to be infected. Subsequently, the inflamed skins were aseptically removed and chopped, and then phenol-added glycerin water was added. The mixture was ground with a homogenizer to be emulsified. Subsequently, the emulsion was filtered. The obtained filtrate was adjusted to weak acidity (pH 4.5 to 5.5) with hydrochloric acid, then heated at 100°C and filtered. The filtrate was adjusted to weak alkalinity (pH 8.5 to 10.0) with sodium hydroxide, further heated at 100°C and then filtered. The filtrate was adjusted to about pH 4.5 with hydrochloric acid, and about 1.5% activated carbon was added. The mixture was stirred for 1 to 5 hours and then filtered. To the activated carbon collected by the filtration, water was added. The mixture was adjusted to pH 9.4 to 10 with sodium hydroxide, stirred for 3 to 5 hours, and then filtered. The filtrate was neutralized with hydrochloric acid.

### Example 3

Skins of healthy adult rabbits were inoculated with vaccinia virus to be activated. Then the activated skins were aseptically removed and chopped, and water was added. The mixture was ground with a homogenizer to be emulsified. Subsequently, the emulsion was filtered under pressure. The obtained filtrate was adjusted to pH 5.0 with hydrochloric acid, and then heated at 100°C with flowing steam. After deproteinization by filtration, the filtrate was adjusted to pH 9.1 with sodium hydroxide, further heated at 100°C and then filtered. The filtrate was adjusted to pH 4.1 with hydrochloric acid, and 2% activated carbon was added. The mixture was stirred for 2 hours and then filtered. To the filtrate, 5.5% activated carbon was further added, and the mixture was stirred for 2 hours and then filtered. To the activated carbon collected by the former filtration, water was added. The mixture was adjusted to pH 9.9 with sodium hydroxide, stirred at 60°C for 1.5 hours, and then filtered. To the former activated carbon and the latter activated carbon, water was added. The mixture was adjusted to pH 10.9 with sodium hydroxide, stirred at 60°C for 1.5 hours, and then filtered. The filtrates were combined and neutralized with hydrochloric acid. Then, the filtrate was desalted by electrodialysis using a membrane with a molecular weight of 100, and dried under reduced pressure.

Now there will be shown an example of the results of pharmacological test concerning the preventive and therapeutic action for muscle injury where the extract from inflamed tissues inoculated with vaccinia virus of the present invention (hereinafter, it will be sometimes referred to as "the present extract") prepared in the above Example 1 was used as a test drug.

### Pharmacological test

Twenty-four 8-week-old male SD (Sprague-Dawley) rats were used. In each of the rats, a right side of the body relative to the spinous process that served as the center was defined as an injury side. Each of the rats was subjected to general anesthesia (0.3 to 0.5 mL of somnopentyl diluted into a double volume with physiological saline was administered intraperitoneally), and was then fixed to a testing bench by fastening rubber bands respectively around four legs thereof. An injury-scheduled site (right paraspinal muscle, the segment was a fourth lumbar vertebra level) was well shaved. A plastic-made cylindrical tip was arranged on the injury-scheduled site, and a heavy bob having a weight of 115 g was dropped from a height of 1 m (the heavy bob was free-fall dropped in the plastic-made cylinder) to cause the injury of paraspinal muscle [the drop mass method described in a reference document (Crisco JJ, Joki P, Heinen GT, Connell MD, Panjabi MM. A muscle contusion injury model. Biomechanics, physiology, and histology. Am J Sports Med. 1994;22(5):702-10.) was partly modified]. Immediately after the damaging, the rats were divided into a group in which physiological saline (5 µL) was subcutaneously injected to the injury site (an untreated group: n = 12) and a group in which the extract of the invention (5 µL) was subcutaneously injected to the injury site (a treated group: n = 12), and the evaluation was carried out on each of the groups.

The evaluation items were those which relate to an acute phase reaction and a tissue repair reaction in the staining of the injured paraspinal muscle with hematoxylin-eosin (HE) in each group (n = 4) on day 1, day 3 and day 7 after the injury, and comparative examination on the evaluation items was carried out.

In each time course, each of the rats was subjected to general anesthesia (0.3 to 0.5 mL of somnopentyl diluted into a double volume with physiological saline was administered intraperitoneally), then paraspinal muscle was excised from the injury site, and then the excised piece was subjected to tissue fixation with a 4% paraformaldehyde (PFA) solution. The specimen that was subjected to the 4% PFA fixation was dehydrated with ethyl alcohol using Tissue-Tek [registered trademark] VIP (manufactured by Sakura Finetek Japan Co., Ltd.) to prepare a paraffin block, and the paraffin block was installed in a microtome SM2010R (manufactured by Leica microsystems, Wetzlar, Germany) to produce a paraffin slice slide having a thickness of about 4 µm. The HE staining was carried out in the following order: deparaffinization (three xylene vessels: 3 minutes and 30 seconds in total → four alcohol vessels: 5 minutes in total); washing with running water (3 minutes); washing with distilled water (1 minute); staining of nucleus (Mayer's hematoxylin solution (hematoxylin I: 20 seconds → hematoxylin II: 3 minutes)); washing with running water (3 minutes); development of color with phosphate buffered saline; washing with running water (1 minute); 50% alcohol (20 seconds); staining of a cytoplasm or the like (eosin: 30 seconds); dehydration (five 100% alcohol vessels: 4 minutes in total); clearing (four xylene vessels: 4 minutes in total); and mounting. Using the treated slide, the observation was carried out with respect to the above-mentioned evaluation items in the injury site using a microscope (BX51, manufactured by Olympus Corporation).

### Results

As the method for evaluating the HE staining, the relative level (amount) of each of the observation items, i.e., the evidence of an acute phase reaction such as edema and bleeding, the proliferative evidence (initiation of tissue repair) such as neutrophil aggregation and fibroblasts appearance and the evidence (tissue delayed union) of fatty degeneration and muscle fiber atrophy, was rated in accordance with the following criteria and was scored: 0 point: the evidence was not observed; 1 point: the evidence was observed extremely slightly; 2 points: the evidence was observed moderately; and 3 points: the evidence was observed at a high degree.

The results of the average scores of paraspinal muscle HE staining evaluation on each group in each of time courses are shown in the following tables

**[Table 1]**

| Table 1 : On day 1 after the injury | | | | | |
|---|---|---|---|---|---|
| | Edema | Bleeding | Neutrophil aggregation | Fibroblast appearance | Fatty degeneration |
| | | | | | Muscle atrophy |
| Untreated group (a verage±SD) | 2.25±0.96 | 2.50±0.58 | 0.25±0.50 | 0.50±0.58 | 0.25±0.50 |
| Treated group (a verage±SD) | 1.75±0.50 | 2.00±0.00 | 0.50±0.58 | 0.25±0.50 | 0.00±0.00 |

On day1 after the injury, in the treated group, the scores of edema and bleeding were lower compared with those in the untreated group, and it was demonstrated that the occurrence of edema and bleeding in an acute phase was suppressed.

**[Table 2]**

| Table 2 : On day 3 after the injury | | | | | |
|---|---|---|---|---|---|
| | Edema | Bleeding | Neutrophil aggregation | Fibroblast appearance | Fatty degeneration |
| | | | | | Muscle atrophy |
| Untreated group | 2.00±0.82 | 2.00±0.82 | 1.75±0.50 | 1.75±0.50 | 0.50±0.58 |
| (a verage±SD) | | | | | |
| Treated group (a verage±SD) | 1.50±0.58 | 1.5±1.00 | 2.75±0.50 | 2.5±0.58 | 1.00±0.00 |

On day 3 after the injury, in the treated group, the scores of neutrophil aggregation and appearance of fibroblasts were higher compared with those in the untreated group, and it was demonstrated that the tissue repair process was initiated more surely.

**[Table 3]**

| Table 3 : On day 7 after the injury | | | | | |
|---|---|---|---|---|---|
| | Edema | Bleeding | Neutrophil aggregation | Fibroblast appearance | Fatty degeneration |
| | | | | | Muscle atrophy |
| Untreated group (a verage±SD) | 1.50±0.58 | 2.00±0.82 | 2.75±0.50 | 2.75±0.50 | 2.75±0.50 |
| Treated group (a verage±SD) | 0.25±0.50 | 0.50±0.58 | 0.50±0.58 | 0.50±0.58 | 0.25±0.50 |

On day 7 after the injury, in the treated group, the scores of fatty degeneration and muscle atrophy were lower compared with those in the untreated group. Therefore, the tendency of a tissue delayed union was not observed, and it was demonstrated that a good tissue repair process (absorption of denatured cell tissues, repair of muscle fibers, and the like) proceeded.

From the results shown in Tables 1 to 3 above, in the treated group, the suppression of edema and bleeding on day 1 after the injury, the secured initiation of the tissue repair process on day 3 after the injury, and the progression of the good tissue repair process on day 7 after the injury were observed when compared with the untreated group. That is, it was demonstrated that the extract of the invention had a repair promotive effect on muscle injury.

### [Industrial Applicability]

As will be apparent from the above results of the pharmacological test, since it was confirmed that the medicament of the present invention had repair promotive effect on muscle injury, the medicament of the present invention has been shown to be useful as a preventing or therapeutic agent for muscle injury. A commercially available extract from inflamed skin inoculated with vaccinia virus has been used for many years and has been recognized to be a medicinal agent having very high safety. As such, the medicament of the present invention is effective as a preventing or therapeutic agent for muscle injury and is very highly useful with high safety and few side effects.

## Claims

1. An extract from inflamed tissue of rabbit skin, which tissue has been inflamed by inoculation with vaccinia virus, for use in treating a muscle injury; which extract is obtainable by crushing said inflamed tissue, adding an extraction solvent to remove tissue fragments, deproteinising the solution, adsorbing the deproteinised solution onto an adsorbent, and eluting the extract.

2. An extract for use according to claim 1, wherein the muscle injury is associated with surgical invasion.

3. An extract for use according to claim 1 or claim 2, wherein the extract is formulated as an injectable preparation.

4. An extract from inflamed tissue of rabbit skin, which tissue has been inflamed by inoculation with vaccinia virus, for use in promoting a muscle injury repair; which extract is obtainable by crushing said inflamed tissue, adding an extraction solvent to remove tissue fragments, deproteinising the solution, adsorbing the deproteinised solution onto an adsorbent, and eluting the extract.

5. An extract for use according to claim 4, wherein the muscle injury is associated with surgical invasion.

6. An extract for use according to claim 4 or claim 5, wherein the extract is formulated as an injectable preparation.

## Patentansprüche

1. Extrakt aus entzündetem Hasenhautgewebe, wobei das Gewebe durch Impfen mit Vacciniavirus entzündet wurde, zur Verwendung in der Behandlung einer Muskelverletzung; wobei das Extrakt durch das Mahlen des entzündeten Gewebes, Hinzufügen eines Extraktionslösemittels zum Entfernen der Gewebefragmente, Deprotonisierung der Lösung, Adsorbieren der deprotonisierten Lösung auf ein Adsorptionsmittel, und Herauslösen des Extrakts gewonnen werden kann.

2. Extrakt zur Verwendung nach Anspruch 1, wobei die Muskelverletzung mit einem operativen Eingriff verbunden ist.

3. Extrakt zur Verwendung nach Anspruch 1 oder 2, wobei das Extrakt als ein injizierbares Präparat formuliert ist.

4. Extrakt aus entzündetem Hasenhautgewebe, wobei das Gewebe durch Impfen mit Vacciniavirus entzündet wurde, zur Verwendung in der Förderung einer Muskelverletzungsheilung; wobei das Extrakt durch das Mahlen des entzündeten Gewebes, Hinzufügen eines Extraktionslösemittels zum Entfernen der Gewebefragmente, Deprotonisierung der Lösung, Adsorbieren der deprotonisierten Lösung auf ein Adsorptionsmittel, und Herauslösen des Extrakts gewonnen werden kann.

5. Extrakt zur Verwendung nach Anspruch 4, wobei die Muskelverletzung mit einem operativen Eingriff verbunden ist.

6. Extrakt zur Verwendung nach einem der Ansprüche 4 oder 5, wobei das Extrakt als ein injizierbares Präparat formuliert ist.

## Revendications

1. Extrait de tissu de peau de lapin enflammé, ledit tissu a été enflammé par inoculation avec le virus de la vaccine, pour une utilisation dans le traitement d'une lésion musculaire ; ledit extrait peut être obtenu par écrasement dudit tissu enflammé, ajout d'un solvant d'extraction pour prélever des fragments de tissu, déprotéinisation de la solution, adsorption de la solution déprotéinisée sur un adsorbant et élution de l'extrait.

2. Extrait pour une utilisation selon la revendication 1, dans lequel la lésion musculaire est associée à une invasion chirurgicale.

3. Extrait pour une utilisation selon la revendication 1 ou la revendication 2, dans lequel l'extrait est formulé sous forme de préparation injectable.

4. Extrait provenant de tissu de peau de lapin enflammé, ledit tissu a été enflammé par inoculation avec le virus de la vaccine, pour une utilisation dans la stimulation d'une cicatrisation de lésion musculaire ; ledit extrait peut être obtenu par écrasement dudit tissu enflammé, ajout d'un solvant d'extraction pour prélever des fragments de tissu, déprotéinisation de la solution, adsorption de la solution déprotéinisée sur un adsorbant et élution de l'extrait.

5. Extrait pour une utilisation selon la revendication 4, dans lequel la lésion musculaire est associée à une invasion chirurgicale.

6. Extrait pour une utilisation selon la revendication 4 ou la revendication 5, dans lequel l'extrait est formulé sous forme de préparation injectable.
